# EUROPÄISCHE PATENTSCHRIFT

(11) **EP 3 881 881 B1**
(45) Veröffentlichungstag und Bekanntmachung des Hinweises auf die Patenterteilung: **02.12.2026**
(21) Anmeldenummer: 20163366.6
(22) Anmeldetag: 16.03.2020
(51) Int. Cl.: A61M 5/32

(54) **ZUSATZFUNKTION EINER SICHERHEITSVORRICHTUNG FÜR SPRITZEN**
ACCESSORY FUNCTION OF A SAFETY DEVICE FOR SYRINGES
FONCTION SUPPLÉMENTAIRE D'UN DISPOSITIF DE SÉCURITÉ POUR SERINGUES

(43) Veröffentlichungstag der Anmeldung: 22.09.2021
(73) Patentinhaber: Gerresheimer Regensburg GmbH, 93047 Regensburg (DE)
(72) Erfinder: Leuschner, Udo, 93049 Regensburg (DE); Großer, Jörg, 93138 Lappersdorf (DE)
(74) Vertreter: Fish & Richardson P.C.

(56) Entgegenhaltungen:
- EP-A1- 2 572 745
- EP-A1- 3 106 193
- EP-A1- 3 106 194
- US-B2- 9 731 081

## Beschreibung

Die vorliegende Erfindung betrifft eine Sicherheitsvorrichtung für Spritzen, ein Verfahren zur Montage einer Sicherheitsvorrichtung, ein Spritzensicherungssystem sowie eine Spritze ausgestattet mit einer Sicherungsvorrichtung.

Bekanntermaßen wird beim Spritzensicherungssystem der Kragen mit seinen Pins, beispielsweise Führungsstiften, in einer Kurvenführung des Hülsenelements gegen eine Federkraft bis zu einem Anschlag bewegt. Die Kanüle steht aus dem Hülsenelement heraus. Somit kann eine Injektion verabreicht werden. Wird die Spritze vom Anwender abgesetzt, entspannt sich die Feder wieder. Dabei wird der Kragen mit seinen Pins in eine Verriegelungsposition bewegt. Die Spritze kann nicht wiederverwendet werden. Sollte der Injektionsvorgang abgebrochen werden, nachdem die Kanülenspitze das Hülsenelement verlassen hat, soll das Sicherungssystem eine Wiederverwendung verhindern. Der Kragen soll auch hier durch die Federkraft in die Verriegelungsposition bewegt werden. Die Kurvenführung beschreibt nicht nur eine Bewegungsrichtung in vertikaler Richtung, sondern auch in horizontaler Richtung. Deshalb wird die Feder gespannt und tordiert. Aufgrund der Torsion wird der Kragen beim Entspannen der Feder wieder in Richtung "Ausgangsstellung" rotiert. Das Sicherungssystem kann somit wieder in Ausgangsstellung gehen und wiederverwendet werden.

Des Weiteren beschreibt beispielsweise die DE 10 2016 108 870 A1 eine Stechmittelschutzvorrichtung, die sich dadurch auszeichnet, dass ein mit dem Hülsenelement verbundenes Verbindungselement zumindest einen sich in axialer Richtung erstreckenden Vorsprung aufweist, welcher zumindest abschnittsweise in einer Ausnehmung des inneren Elements aufgenommen ist, so dass eine kraft- und / oder formschlüssige Verbindung zwischen dem Hülsenelement und dem inneren Element besteht.

DE 10 2015 110 343 A1 beschreibt eine Sicherheitsvorrichtung, die sich dadurch auszeichnet, dass diese ein bei einer Anwendung der Spritze die Haut eines Patienten kontaktierendes Kontaktelement umfasst, welches an dem distalen Ende des Hülsenelements angeordnet ist und unabhängig von dem Hülsenelement rotierbar ist.

DE 10 2015 111 835 A1 beschreibt eine Sicherheitsvorrichtung, die sich dadurch auszeichnet, dass die Öffnungsfläche zumindest abschnittsweise einen Teilbereich aufweist, der einen Winkel α mit der axialen Richtung einschließt, wobei der Winkel α in einem Bereich zwischen 10° und 80° liegt.

DE 10 2015 111 840 A1 beschreibt eine Sicherheitsvorrichtung, die sich dadurch auszeichnet, dass das innere Element einen Kragenabschnitt und einen das Stechmittel umschließenden Stechmittelschutzabschnitt aufweist, wobei der Kragenabschnitt an einem distalen Endbereich des Spritzenkörpers anordenbar ist und die Sicherheitsvorrichtung an dem Spritzenkörper befestigt und der Stechmittelschutzabschnitt vor Anwendung der Spritze von dem Kragenabschnitt lösbar ist.

EP 3 106 191 A1 beschreibt eine Sicherheitsvorrichtung, die sich dadurch auszeichnet, dass das Kragenelement in einer Umfangsrichtung rotierbar an dem distalen Endbereich des Spritzenkörpers angeordnet ist und zumindest ein Toleranzausgleichselement aufweist, welches mit dem Spritzenkörper mechanisch in Wirkkontakt ist, so dass Toleranzen des Spritzenkörpers ausgleichbar sind.

EP 3 106 193 A1 beschreibt einen Spritzenkörper mit einer endseitig angeordneten Sicherheitseinrichtung zur Vermeidung von Stichverletzungen, wobei der Spritzenkörper ein endseitig angeordnetes Stechelement umfasst und die Sicherheitseinrichtung eine Ausnehmung und mindestens einen Führungsstift aufweist, mittels welcher eine Führungskulisse ausgebildet ist, um den Führungsstift bei einer Relativbewegung von Spritzenkörper zu Sicherheitseinrichtung in einer Längsrichtung des Spritzenkörpers zu führen, wobei die Führungskulisse einen ersten und einen zweiten Kulissenbereich umfasst, die durch eine in einer Längsrichtung des Spritzenkörpers verlaufenden fiktiven Trennlinie voneinander getrennt sind, wobei der Führungsstift in einer Ausgangsstellung in dem ersten Kulissenbereich anordenbar ist und von dem ersten in den zweiten Kulissenbereich in eine Endstellung durch Überschreiten der Trennlinie überführbar ist, wenn ein distales Ende des Stechelements bei der Relativbewegung von Spritzenkörper zu Sicherheitseinrichtung auf der Höhe einer Austrittsöffnung der Sicherheitseinrichtung angeordnet ist.

EP 3 106 194 A1 beschreibt eine Sicherheitsvorrichtung, sie sich dadurch auszeichnet, dass die Sicherheitsvorrichtung ein Kappenelement aufweist, welches zumindest abschnittsweise über dem Hülsenelement anordenbar ist und durch welches das Hülsenelement bezüglich der Relativbewegung des Spritzenkörpers zu dem Hülsenelement arretierbar ist.

EP 2 572 745 A1 beschreibt eine Nadelsicherheitsvorrichtung mit einer Nadelnabe, die einen radialen Vorsprung aufweist, eine Nadel, die mit der Nadelnabe gekoppelt ist und eine distale Spitze hat, und eine innere Hülse, die teleskopisch mit der Nadelnabe gekoppelt ist. Die innere Hülse umfasst eine erste Spur, die so angepasst ist, dass sie mit dem radialen Vorsprung in Eingriff gelangen kann. Die erste Spur umfasst einen axialen Abschnitt und einen radialen Abschnitt. Wenn sich die innere Hülse in einer ersten vorgeschobenen Position und einer ersten Winkelstellung relativ zu der Nadelnabe befindet, ist der radiale Vorsprung in der ersten Bahn in Eingriff und ist die distale Spitze abgedeckt. Wenn sich die innere Hülse in einer zurückgezogenen Position relative zu der Nadelnabe befindet, ist der radiale Vorsprung in dem axialen Abschnitt und die distale Spitze liegt frei. Wenn sich die innere Hülse in einer zweiten vorgeschobenen Position relativ zu der Nadelnabe und einer zweiten Winkelstellung befindet, ist der radiale Vorsprung in dem radialen Abschnitt und ist die distale Spitze abgedeckt.

US 9 731 081 B2 beschreibt eine Nadelschutzvorrichtung, die an einer vorgefüllten Spritze in ihrem füllfertigen Zustand montiert werden kann. Die Vorrichtung umfasst einen Vorrichtungsschutz, der mit einem Verriegelungskragen mit einem flexiblen Element verbunden ist und so vorgespannt ist, dass er sich relativ zu dem Verriegelungskragen bewegt. Der Verriegelungskragen ist mit dem Spritzenhals verbunden, um die Vorrichtung an der Spritze zu befestigen. Wenn sich der Vorrichtungsschutz proximal bewegt, wirken Dreharme des Verriegelungskragens mit abgewinkelten Ausschnitten in dem Vorrichtungsschutz zusammen, wodurch bewirkt wird, dass sich der Vorrichtungsschutz relativ zu dem Verriegelungskragen dreht und eine oder mehrere Führungsnasen an dem Vorrichtungsschutz aus einer oder mehreren Führungsnuten in dem Verriegelungskragen gelöst werden, wodurch veranlasst wird, dass sich der Vorrichtungsschutz aus einer ersten Konfiguration, in der der Vorrichtungsschutz zurückziehbar ist, um eine Spritzenspitze freizulegen, in eine zweite Konfiguration bewegt, in der der Vorrichtungsschutz fest positioniert ist, um die Spritzenspitze abzudecken.

Aufgabe der Erfindung ist es somit, ein Wiederverwenden der Spritze beziehungsweise der Nadel zu verhindern, nachdem die Nadel oder Kanülenspitze aus dem Hülsenelement passiert ist beziehungsweise nachdem der Führungsstift des Kragenelementes eine gewisse Position auf der Kurvenbahn / Führungskulisse des Hülsenelementes erreicht hat.

Eine weitere Aufgabe der vorliegenden Erfindung ist es, das Risiko des Zurückspringens des Kragenelementes in die Ausgangsstellung zu vermindern oder ganz zu vermeiden. Gelöst werden diese Aufgaben mit den Merkmalen der Ansprüche.

Erfindungsgemäß ist das Hülsenelement und das Kragenelement der Sicherheitsvorrichtung jeweils mit einem Verrastmechanismus vorgesehen.

Dabei weist der Verrastmechanismus des Hülsenelements zumindest eine Rippe oder Welle auf, die vertikal bzw. longitudinal entlang der Spritzenlänge angeordnet ist.

Vorzugsweise weist das Hülsenelement zwei gegenüberliegende Rippen auf. Vorteilhafterweise wird das Kragenelement über die Rippen des Hülsenelementes beim Drücken gegen eine Federkraft durch den Verrastmechanismus geschnappt. Dadurch wird eine Fixierung in eine Rotationsrichtung bewirkt.

Die Rippen oder Wellen weisen Flanken mit unterschiedlichen Flankenwinkeln auf, die derart ausgebildet sind, dass der eine Flankenwinkel flach und der andere Flankenwinkel steil ist. Am distalen Ende des Hülsenelementes befindet sich eine Austrittsöffnung. Das proximale Ende des Hülsenelementes ist mit dem Spritzenkörper verbunden.

Bevorzugt weist der Verrastmechanismus des Kragenelements zumindest eine Ausbuchtung auf seiner Mantelfläche mit Flanken auf, wobei die Flanken unterschiedliche Flankenwinkel aufweisen, wobei der eine Flankenwinkel flach und der andere Flankenwinkel steil ausgebildet ist.

Bevorzugt rotieren das Kragenelement und das Hülsenelement unmittelbar an den Rippen und Ausbuchtungen nur in eine Richtung, beispielsweise vergleichbar mit einer Ratschenfunktion. Durch die Verrastung des Verrastmechanismus des Kragens und Hülsenelementes wird die Sicherheit vor einer Wiederverwendung der Nadelspitze gewährleistet.

Der Innendurchmesser des Hülsenelementes und der Außendurchmesser des Kragenelementes überdecken sich in den Bereichen der Ausbuchtungen des Kragenelementes und der Rippen des Hülsenelementes. Bevorzugt wird ein Bereich des Außendurchmessers des Kragenelementes durch den Innendurchmesser des Hülsenelementes nicht überdeckt, so dass die Rippen des Hülsenelementes freistehen.

Die Sicherheitsvorrichtung weist mindestens ein Federelement auf, das mit dem Spritzenkörper wirkverbunden ist und der Relativbewegung des Hülsenelementes zu der Sicherheitsvorrichtung entgegenwirkt, wobei das Kragenelement beim Drücken gegen die Federkraft durch die Sicherheitsvorrichtung aufgrund der vorgegebenen Führungskulisse rotiert und über die Rippen des Hülsenelementes durch den Verrastmechanismus schnappt.

Aufgrund der Flankenwinkel des Hülsenelementes und des Kragenelementes ist eine Rotation des Kragenelementes in dem Bereich der Führungskulisse, der in eine Ausgangsstellung führt, ausgeschlossen. Das Kragenelement befindet sich in der Ausgangsstellung an einem proximalen Ende des Hülsenelementes. Die Austrittsöffnung befindet sich an einem distalen Ende des Hülsenelementes.

Bevorzugt wird das Kragenelement in dem Bereich der Führungskulisse gehalten, der in der Arretierung des Kragenelementes zum Hülsenelement ausläuft. Durch die ausgeübte Federkraft auf das Kragenelement sowie den ausgebildeten Rippen an dem Hülsenelement und den Ausbuchtungen an dem Kragenelement werden die Ausbuchtungen des Kragenelementes in die Rippen des Hülsenelementes geführt und geschnappt. Das Hülsenelement weist eine Führungskulisse auf, in welcher mindestens ein Führungsstift läuft, wodurch verschiedene Positionen des Hülsenelements realisiert werden können. Dabei findet das Schnappen der Ausbuchtungen in den Rippen in einer Position der Führungskulisse statt, wenn die Nadelspitze soweit in dem Hülsenelement in distaler Richtung verschoben wurde, so dass eine Kontamination der Nadelspitze nicht mehr ausgeschlossen werden kann. Beim Zurückführen des Führungsstiftes in der Führungskulisse Richtung proximales Ende des Hülsenelementes wird der Führungsstift durch die Ausgestaltung der Führungskulisse in der Endposition gehalten. So wird verhindert, dass das Kragenelement in die Ausgangsposition zurückspringt und die Nadel evtl. erneut zum Einsatz kommt, wenn sich das Kragenelement wieder zum proximalen Ende des Hülsenelementes bewegt.

Dadurch wird ein Wiederverwenden der Spritze verhindert. Und durch den Verrastmechanismus des Hülsen- und Kragenelementes ist somit eine zusätzliche Sicherheit vor Wiederverwendung der Spritze gewährleistet.

Gemäß einer Ausführungsform ist das Kragenelement im Wesentlichen als hohler Kreiszylinder ausgebildet. Bevorzugt weist der Kreiszylinder eine Mantelfläche auf, an der der zumindest eine Führungsvorsprung angeordnet ist. Vorzugsweise erstreckt sich der zumindest eine Führungsvorsprung radial von der Mantelfläche weg. Weiterhin bevorzugt ist der Führungsvorsprung als Kreiszylinder beziehungsweise als Stift ausgebildet. Vorteilhafterweise sind an der Mantelfläche zwei sich diametral gegenüberliegende Führungsvorsprünge angeordnet. Demzufolge würde auch das Hülsenelement zwei sich diametral gegenüberliegende Führungskulissen aufweisen, in denen jeweils ein Führungsvorsprung geführt wird. Erfindungsgemäß ist das Kragenelement weiterhin in einer Umfangsrichtung rotierbar an dem distalen Endbereich des Spritzenkörpers angeordnet. Bei einer Anwendung der Spritze wird die Spritze mit der Sicherheitsvorrichtung gegen die Haut des Patienten gedrückt. Durch die Relativbewegung des Spritzenkörpers zu dem Hülsenelement und die Führung des Führungsvorsprungs in der Führungskulisse wird eine Rotation des Kragenelements entlang einer Umfangsrichtung verursacht. Das Hülsenelement schiebt sich dadurch über den Spritzenkörper, wodurch das Stechmittel, welches eine Kanüle, eine Nadel oder auch eine Lanzette sein kann, durch eine entsprechende Öffnung in dem Hülsenelement hindurchtritt. Somit wird eine Rotation des Hülsenelements auf der Haut des Patienten um die Einstichstelle herum vermieden.

Gemäß der Erfindung weist das Kappenelement eine Stechmittelschutzeinrichtung auf, in welcher das Stechmittel anordenbar ist. Durch eine derartige Stechmittelschutzeinrichtung wird ein weiterer Schutz des Stechmittels vor Beschädigungen und insbesondere vor Kontamination gewährleistet.

Vorzugsweise weist das Hülsenelement eine distale Öffnung auf. Bevorzugt ist dabei der Innendurchmesser der distalen Öffnung zumindest abschnittsweise größer als der Außendurchmesser der Stechmittelschutzeinrichtung, so dass die Stechmittelschutzeinrichtung innerhalb des Hülsenelements anordenbar ist.

Erfindungsgemäß ist die Stechmittelschutzeinrichtung mit dem Kragenelement in Wirkkontakt bringbar, wodurch das Kragenelement bezüglich einer Rotation arretierbar ist. Ein solcher Wirkkontakt kann beispielweise ein reibschlüssiger Kontakt sein. Es wäre aber auch denkbar, dass die Stechmittelschutzeinrichtung und das Kragenelement zueinander korrespondierende Rasteinrichtungen aufweisen, welche eine Rotation des Kragenelements verhindern.

Gemäß einer bevorzugten Ausführungsform weisen das Kappenelement und das Hülsenelement zueinander komplementäre Rastelemente auf, so dass das Kappenelement und das Hülsenelement trennbar verrastbar sind. Es wäre vorstellbar, dass ein Rastelement eine Sollbruchstelle aufweist, welche vor der Anwendung aufgebrochen werden muss, um ein Abziehen des Kappenelements von dem Hülsenelement zu ermöglichen. Es wäre aber auch denkbar, dass ein Einrasten der Rastelemente auch nach der Anwendung der Spritze ermöglicht ist. Somit wäre es ermöglicht, dass Kappenelement nach dem Gebrauch der Spritze wieder fest auf dem Hülsenelement anzuordnen, wodurch das Hülsenelement bezüglich der Relativbewegung des Spritzenkörpers zu dem Hülsenelement wiederum arretierbar wäre. Demzufolge ist eine gefahrlose Entsorgung der gebrauchten Spritze möglich, welche somit kein Verletzungsrisiko mehr darstellt.

Gemäß einer weiteren Ausführungsform der Erfindung umfasst das Kappenelement zumindest ein flügelartiges Element, welches in zumindest einer Aufnahme des Hülsenelements aufnehmbar ist. Weiterhin bevorzugt weist das Kappenelement zwei flügelartige Elemente auf, welche diametral in jeweils zwei Aufnahmen des Hülsenelements aufnehmbar sind. Besonders bevorzugt sind die beiden flügelartigen Elemente diametral sich gegenüberliegend an dem Kappenelement angeordnet.

Nach einer weiteren bevorzugten Ausführungsform ist zumindest ein flügelartiges Element mit dem Kragenelement in Wirkkontakt bringbar, wodurch das Kragenelement bezüglich einer Rotation arretierbar ist. Ein solcher Wirkkontakt kann beispielweise ein reibschlüssiger Kontakt sein. Es wäre aber auch denkbar, dass die Stechmittelschutzeinrichtung und das Kragenelement zueinander korrespondierende Rasteinrichtungen aufweisen, welche eine Rotation des Kragenelements verhindern.

Gemäß einer weiteren Ausführungsform ist an dem zumindest einen flügelartigen Element des Kappenelements zumindest ein Rastelement angeordnet, welches in zumindest ein komplementäres Rastelement, das in der zumindest einen Aufnahme des Hülsenelements angeordnet ist, einrastbar ist.

Vorzugsweise ist das Kappenelement integral mit der Stechmittelschutzeinrichtung ausgebildet. Eine derartige Ausführung der Sicherheitsvorrichtung hat eine kostengünstige und einfache Herstellung zum Vorteil.

Es ist aber auch denkbar, dass das Kappenelement eine distale Öffnung aufweist, wobei die distale Öffnung als Aufnahme ausgebildet ist, um die Stechmittelschutzeinrichtung aufzunehmen. Eine solche Ausgestaltung ermöglicht es, das Kappenelement und die Stechmittelschutzeinrichtung aus unterschiedlichen Materialien herzustellen. Es wäre demnach denkbar, die Stechmittelschutzeinrichtung aus einem elastischen Material, beispielsweise Gummi herzustellen. Ein solches elastische Material begünstigt eine Reduzierung des Beschädigungsrisikos des Stechmittels.

Vorzugsweise weist die Sicherheitsvorrichtung mindestens ein Federelement auf, das mit dem Spritzenkörper wirkverbunden ist und der Relativbewegung des Spritzenkörpers zu der Sicherheitsvorrichtung entgegenwirkt. Demnach bleibt die Kanüle bis zur vorgesehenen Anwendung innerhalb des Hülsenelements. Bei der Anwendung muss das Hülsenelement gegen die Federkraft verschoben werden, damit die Kanüle durch die Öffnung des Hülsenelements hindurchtreten kann. Nach Gebrauch der Spritze schiebt sich automatisch, angetrieben durch die Federkraft des Federelements, das Hülsenelement wieder über die Kanüle. Durch die Führung des Führungsvorsprungs in der Führungskulisse rotiert das Kragenelement entgegen der Umfangsrichtung. Der Anwender ist somit vor Stichverletzungen mit der gebrauchten kontaminierten Kanüle geschützt. Bevorzugt umfasst das Federelement eine Spiralfeder. Denkbar sind aber auch anderweitige Federarten, wie beispielsweise Schenkelfedern oder Torsionsfedern. Ferner wäre vorstellbar, das Federelement als ein Elastomer auszubilden.

Durch das Federelement kann also gewährleistet werden, dass die Nadel nach Gebrauch der Spritze sicher in die Sicherheitsvorrichtung rückgeführt werden kann und der Führungsstift in die Endstellung vorzugsweise automatisch überführt werden kann.

Dabei kann das Federelement auf verschiedene Weisen ausgestaltet sein. Vorzugsweise handelt es sich bei dem Federelement um eine Spiralfeder.

Gemäß einer bevorzugten Ausführungsform ist die Sicherheitsvorrichtung mittels eines Kragenelements mit dem Spritzenkörper zumindest wirkverbunden. Es ist dabei denkbar, dass der Führungsstift / die Führungsstifte auf dem Kragenelement angeordnet sind. Dabei ist vorteilhaft das Kragenelement einerseits mit einem Nadelaufsatz, der mit dem Spritzenkörper verbunden ist, verbunden und andererseits mittels des zumindest einen Führungsstifts mit der Sicherheitsvorrichtung verbunden, da der Führungsstift innerhalb der Führungskulisse der Sicherheitsvorrichtung angeordnet ist.

Besonders vorteilhaft ist das Kragenelement innerhalb einer Hülse der Sicherheitsvorrichtung angeordnet. Weiter vorteilhaft weist eben diese Hülse auch die Führungskulisse auf. Gemäß einer bevorzugten Ausführungsform umfasst die Sicherheitsvorrichtung, insbesondere die Hülse, zwei Ausnehmungen, und das Kragenelement zwei Führungsstifte, wobei die Ausnehmungen und die Führungsstifte vorteilhaft gegenüberliegend ausgebildet sind, wodurch eine besonders vorteilhafte Führung gewährleistet werden kann.

Weiter bevorzugt ist das Federelement ebenso innerhalb der Hülse angeordnet und besonders bevorzugt mittels des Kragenelements in der Hülse gegen ein Herausfallen gesichert.

Erfindungsgemäß ist ein Verfahren zur Montage einer vorstehend beschriebenen Sicherheitsvorrichtung, auf einem Spritzenkörper, vorgesehen. Das Verfahren umfasst die Schritte:
a. Bereitstellen eines Hülsenelements, eines Federelements und eines Kragenelements (3),
b. Einbringen des Federelements in das Innere des Hülsenelements entlang einer Montagerichtung,
c. Einbringen des Kragenelementes in das Innere des Hülsenelements entlang der Montagerichtung,
d. Erhalten der Sicherheitseinrichtung,
e. Montieren der Sicherheitseinrichtung auf dem Spritzenkörper durch Verbinden des Kragenelements mit dem Spritzenkörper; wobei das Kragenelement zumindest einen Führungsstift aufweist, welcher in zumindest einer Führungskulisse des Hülsenelements eingreift, wobei die Sicherheitsvorrichtung ein Kappenelement aufweist, welches zumindest abschnittsweise über dem Hülsenelement anordenbar ist und durch welches das Hülsenelement bezüglich der Relativbewegung des Spritzenkörpers zu dem Hülsenelement arretierbar ist, wobei das Kragenelement in einer Umfangsrichtung rotierbar an dem distalen Endbereich des Spritzenkörpers angeordnet ist, wobei das Kappenelement eine Stechmittelschutzeinrichtung aufweist, in welcher das Stechmittel anordenbar ist und welche mit dem Kragenelement in Wirkkontakt bringbar ist, wodurch das Kragenelement bezüglich der Rotation arretierbar ist, wobei das Hülsenelement und das Kragenelement jeweils mit einem Verrastmechanismus vorgesehen ist, wobei der Verrastmechanismus des Hülsenelementes zumindest eine Rippe oder Welle aufweist, die longitudinal entlang der Länge des Spritzenkörpers angeordnet ist.

Bevorzugte Ausführungsformen der Erfindung werden nachfolgend anhand der beigefügten Zeichnungen näher erläutert.

Es zeigen:
Fig. 1 - eine perspektivische Ansicht des Kragenelements;
Fig. 2 - eine Draufsicht des Kragenelements;
Fig. 3 - eine perspektivische Ansicht des Hülsenelements;
Fig. 4 - eine perspektivische Ansicht des Hülsenelements mit einem Teilschnitt;
Fig. 5 - eine perspektivische Ansicht des Hülsenelements mit dem Kragenelement;
Fig. 6 - eine perspektivische Ansicht des Hülsenelements und des Kragenelements;
Fig. 7 - eine perspektivische Ansicht des Hülselements und Spritzenkörper;
Fig. 8 - eine perspektivische Ansicht des Hülsenelements mit gedrückter Federkraft;
Fig. 9 - eine perspektivische Ansicht des Hülsenelements mit Spritzenkörper und das herausragende Stechmittel;
Fig. 10 - eine perspektivische Ansicht des Hülsenelements mit Spritzenkörper und das eingezogene Stechmittel;
Fig. 11 - eine perspektivische Ansicht des Hülsenelements in Sicherheitsstellung;
Fig. 12 - ein Spritzenkörper mit darauf montierten Sicherheitsvorrichtung;
Fig. 13 - ein Spritzenkörper mit darauf montierten Sicherheitsvorrichtung und entfernten Kappenelement.

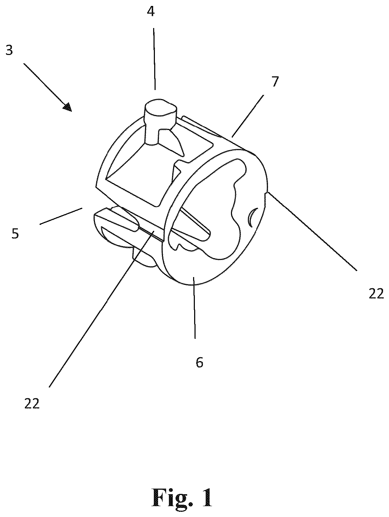

Figur 1 zeigt das Kragenelement 3 mittels welchem das Hülsenelement und somit die Sicherheitsvorrichtung im Ganzen mit dem Spritzenkörper verbindbar ist. Das Kragenelement 3 ist vorliegend im Wesentlichen eine zylindrische Form mit einem Außendurchmesser und einem Innendurchmesser, wobei durch das Bezugszeichen 6 das proximale Ende des Kragenelements bezeichnet ist.

Vorliegend ist auf einer Mantelfläche 7 des Kragenelements 3 ein Führungsstift 4 angeordnet. Dieser Führungsstift 4 ist dann in der Führungskulisse der Hülse anordenbar und mit dieser wirkverbindbar.

Zusätzlich weist das Kragenelement 3 eine oder mehrere, vorliegend zwei, Aussparungen 5 auf.

Bevorzugt ist die Sicherheitsvorrichtung umfassend das Hülsenelement, das Kragenelement 3 und das Kappenelement bereits vormontierbar und als Ganzes mit dem Spritzenkörper mittels des Kragenelements 3 verbindbar.

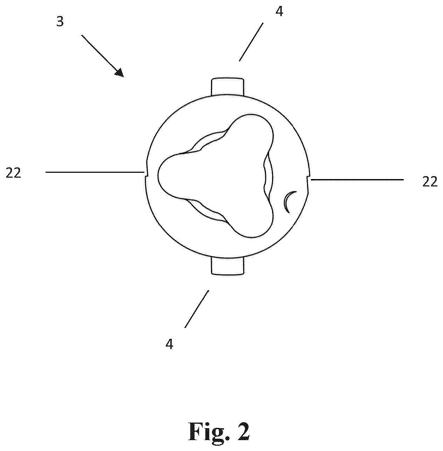

Figur 2 zeigt eine Draufsicht des Kragenelements 3 mit zwei angeordneten Führungsstiften 4 und Ausbuchtungen 22.

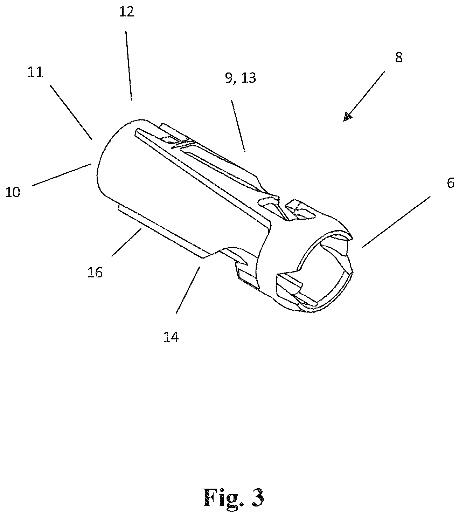

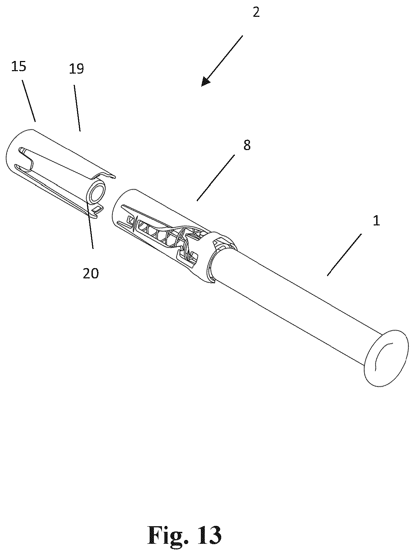

In der Figur 3 wird ein wichtiger Bestandteil der Sicherheitsvorrichtung in einer perspektivischen Ansicht gezeigt, nämlich das Hülsenelement 8. Das Hülsenelement weist eine Ausnehmung 13 auf, die die Führungskulisse 9 zur Führung des Führungsstiftes 4 (hier nicht gezeigt) ausbildet. Zusätzlich kann das Hülsenelement einen Ausnahmebereich 14 aufweisen. Der Ausnahmebereich 14 dient vorliegend dazu, dass ein Kappenelement 15 (siehe Fig. 13) zur Sicherheitserhöhung der Sicherheitsvorrichtung geführt aufsteckbar ist. Durch die Berandung 16 des Ausnahmebereichs 14 ist das Kappenelement mit dem Hülsenelement 8 zumindest in Wirkkontakt und bevorzugt zumindest kraftschlüssig verbunden. Dabei ist der Innendurchmesser 10 des Hülsenelements 8 kleiner als der Außendurchmesser des Kappenelements. Ferner weist das Hülsenelements am distalen Ende 12 vorzugsweise eine Austrittsöffnung 11 auf.

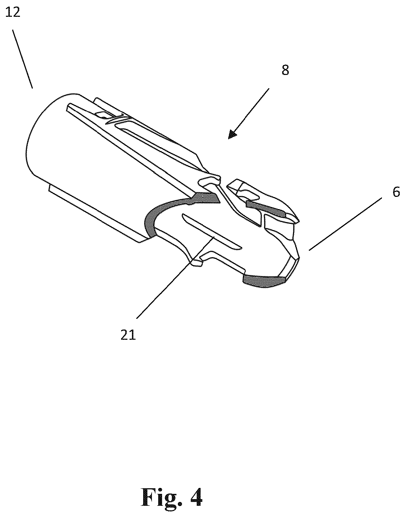

Figur 4 eine perspektivische Ansicht des Hülsenelements mit einem Teilschnitt bzw. ist eine Rippe 21 durch einen Teilschnitt des Hülsenelementes 8 gezeigt. Die Rippe 21 ist dabei vertikal bzw. longitudinal entlang des Spritzenkörpers angeordnet. Die Rippen oder Wellen weisen Flanken mit unterschiedlichen Flankenwinkeln auf, wobei der eine Flankenwinkel flach und der andere Flankenwinkel steil ausgebildet ist. Am distalen Ende 12 des Hülsenelementes 8 befindet sich eine Austrittsöffnung 11. Das proximale Ende 6 des Hülsenelementes 8 ist mit dem Spritzenkörper 1 (nicht in Figur 4 gezeigt) verbunden.

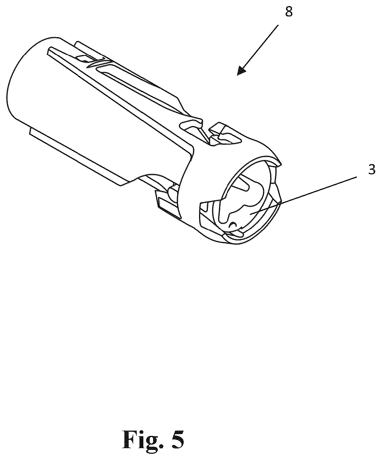

Die Figur 5 zeigt das Hülsenelement 8 mit dem Kragenelement 3. Dabei ist das Kragenelement 3 durch die Führungsstifte mit dem Hülsenelement 8 verbunden.

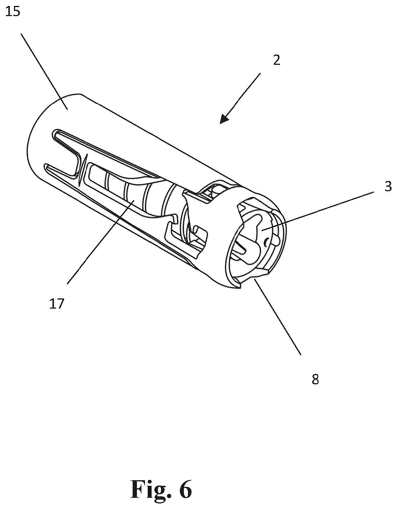

Figur 6 zeigt eine perspektivische Ansicht des Hülsenelements 8 mit dem Kragenelement 3, einem Federelement 17 und einem Kappenelement 15. Das Kappenelement 15 ist näher in Bezug auf Figur 13 beschrieben.

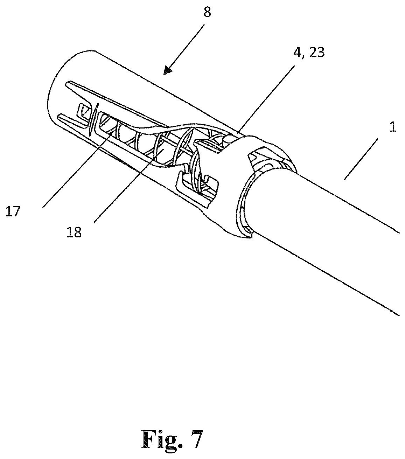

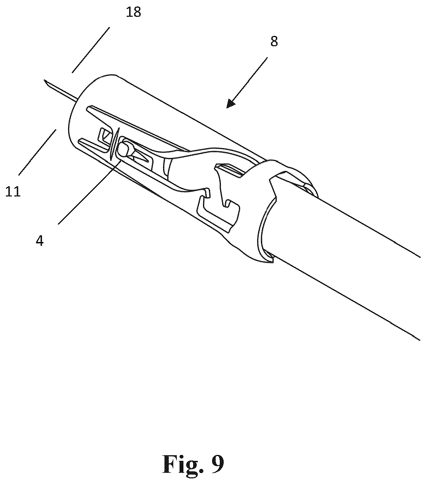

Mit Bezug auf die Figuren 7 bis 11 wird die Sicherheitsvorrichtung 2 im Detail vorgestellt, insbesondere die Bewegung des Führungsstiftes und des Spritzenkörpers 1 relativ zur Sicherheitsvorrichtung 2 sowie die Position des Stechmittels 18. In der Figur 7 ist die Anordnung bestehend aus Spritzenkörper 1 und Sicherheitsvorrichtung 2 in einer Ausgangsstellung zu erkennen. Unter einer Ausgangsstellung wird eine noch nicht benutzte Spritze verstanden. Vorzugsweise wird das distale Ende 12 des Hülsenelements 8 direkt auf die Haut aufgesetzt, so dass die Austrittsöffnung 11 mit der Haut in Kontakt steht. Wird der Spritzenkörper 1 nun relativ zu der Sicherheitsvorrichtung 2 in Längsrichtung bewegt, so wird der Führungsstift durch den Kulissenabschnitt geführt, wodurch das Kragenelement 3 bewegt wird. Im Moment der Überführung ist dabei das distale Ende des Stechmittels 18 auf Höhe der Austrittsöffnung 11 angeordnet. Das Stechmittel 18 ist also gerade im Begriff, aus der Sicherheitsvorrichtung 2 herauszutreten, so dass eine Injektion möglich ist. Das Stechmittel 18 kann dann weiter aus der Sicherheitsvorrichtung 2 herausbewegt werden (siehe Fig. 9). Wurde die Injektion durchgeführt bzw. das Stechmittel 18 aus der Sicherheitsvorrichtung 2 herausbewegt, so vermindert der Benutzer den Druck auf die Spritze, wodurch durch die Federkraft des Federelements der Spritzenköper 1 gegenüber der Sicherheitsvorrichtung 2 entgegen der Längsrichtung bewegt wird. Das Stechmittel wird also durch das Federelement automatisch in die Sicherheitsvorrichtung 2 zurückbewegt.

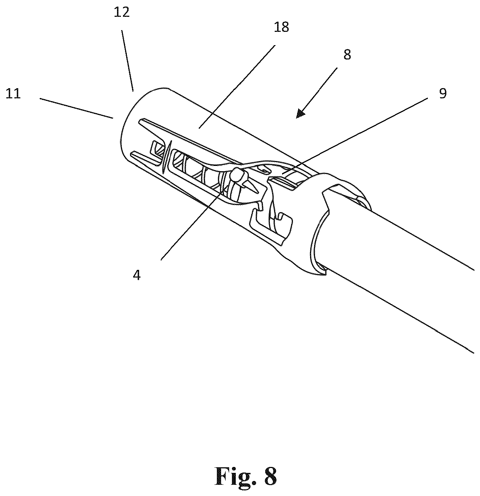

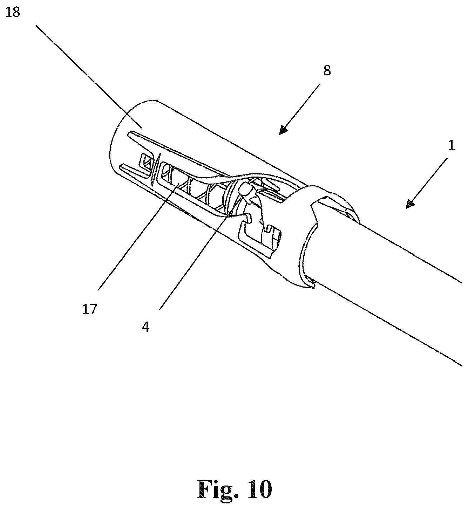

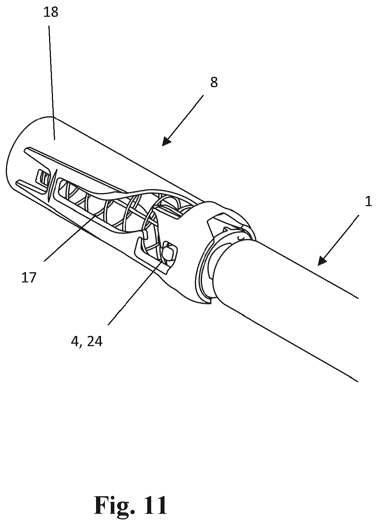

Figur 7 zeigt das Hülsenelement 8, das an seinem proximalen Ende 6 mit dem Spritzenkörper 1 verbunden ist. Das Kragenelement befindet sich in der Ausgangsstellung an einem proximalen Ende des Hülsenelementes bzw. der Führungsstift 4 des Kragenelementes 3 befindet sich in einer Ausgangstellung 23. Die Sicherheitsvorrichtung weist mindestens ein Federelement 17 auf, das mit dem Spritzenkörper wirkverbunden ist und der Relativbewegung des Hülsenelementes 8 zu der Sicherheitsvorrichtung entgegenwirkt, wobei das Kragenelement 3 beim Drücken gegen die Federkraft durch die Sicherheitsvorrichtung aufgrund der vorgegebenen Führungskulisse rotiert. Durch die Ausübung einer Federkraft gegen das Kragenelement 3 bewegt sich das Kragenelement bzw. der Führungsstift 4 in Richtung des distalen Endes 12 bzw. der Austrittsöffnung 11 des Hülsenelementes 8 (gezeigt in Figur 8). Dadurch wird bewirkt, dass das Stechmittel bzw. die Nadel 18 aus der Austrittsöffnung 11 des Hülsenelementes 8 heraustritt (gezeigt in Figur 9). Beim Zurückziehen des Stechmittels 18 bzw. des Kragenelementes, wird der Führungsstift 4 in einer Endposition 24 durch die Ausgestaltung der Führungskulisse 9 verriegelt, so dass das Kragenelement 3 nicht mehr in die Ausgangstellung zurückspringen kann (gezeigt in den Figuren 10 und 11). Durch die Ausgestaltung der Flankenwinkel der Rippen 21 des Hülsenelementes und der Ausbuchtungen 22 des Kragenelementes sowie die ausgeübte Federkraft auf das Kragenelement 3 werden die Ausbuchtungen 22 in die Rippen 21 geführt und geschnappt. Das Hülsenelement weist eine Führungskulisse 9 auf, in welcher mindestens ein Führungsstift 4 läuft, wodurch verschiedene Positionen des Hülsenelements 8 realisiert werden können. Dabei findet das Schnappen der Ausbuchtungen 22 in den Rippen 21 in einer Position der Führungskulisse statt, wenn die Nadelspitze soweit in dem Hülsenelement in distaler Richtung verschoben wurde, so dass eine Kontamination der Nadelspitze nicht mehr ausgeschlossen werden kann. Beim Zurückführen des Führungsstiftes 4 in der Führungskulisse 9 Richtung proximales Ende 6 des Hülsenelementes 8 wird der Führungsstift 4 durch die Ausgestaltung der Führungskulisse in der Endposition 24 gehalten. So wird verhindert, dass das Kragenelement in die Ausgangsposition zurückspringt und die Nadel evtl. erneut zum Einsatz kommt, wenn sich das Kragenelement wieder zum proximalen Ende des Hülsenelementes bewegt.

Dadurch wird ein Wiederverwenden der Spritze verhindert. Und durch den Verrastmechanismus des Hülsen- und Kragenelementes ist somit eine zusätzliche Sicherheit vor Wiederverwendung der Spritze gewährleistet.

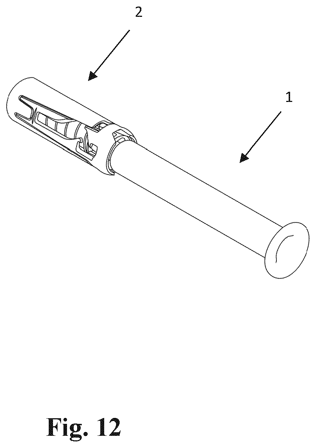

Die Figuren 12 und 13 zeigen einen Spritzenkörper 1 mit einer darauf montierten Sicherheitsvorrichtung 2. Die Sicherheitsvorrichtung 2 umfasst dabei zusätzlich zu dem Kappenelement 15, dem Hülsenelement und dem Kragenelement 3 ein Federelement, das vorliegend als eine Spiralfeder ausgebildet ist. Vorliegend umfasst das Kappenelement 15 auch eine Stechmittelschutzeinrichtung 20 und ein Flügelelement 19, die derart ausgestaltet ist, dass sie komplementär zu dem Ausnahmebereich 14 ausgestaltet ist. Die Stechmittschutzeinrichtung 20 ist vorzugsweise im Wesentlichen zylindrisch ausgestaltet und ist vorteilhaft mit dem Kappenelement 15 fest verbindbar bzw. verbunden, wobei die Stechmittelschutzeinrichtung 20 vorzugsweise derart ausgestaltet ist, dass sie in das Hülsenelement 8 hineinsteckbar ist. Dies bedeutet also, dass ein Außendurchmesser der Stechmittelschutzeinrichtung 20 höchstens dem Innendurchmesser 10 des Hülsenelementes entspricht. Die Dimensionen der Sicherheitsvorrichtung 2 sind derart gewählt, dass das Hülsenelement 8 einen Innendurchmesser 10 aufweist, der größer ist als der Durchmesser des Spritzenkörpers 1, so dass der Spritzenkörper 1 bei einer Bewegung in Längsrichtung nach vorne gegenüber der Sicherheitsvorrichtung 2 der Spritzenkörper in das Hülsenelement 8 hineinbewegbar ist. Gleichzeitig ist der Außendurchmesser des Hülsenelements 8 bzw. der Sicherheitsvorrichtung 2 so gewählt, dass er höchstens einem maximalen Durchmesser einer am proximalen Ende des Spritzenkörpers 1 angebrachten Haltevorrichtung zum Halten und sicheren Setzen der Spritze entspricht.

### Bezugszeichenliste

1: Spritzenkörper
2: Sicherheitsvorrichtung
3: Kragenelement
4: Führungsstift / Führungsvorsprung
5: Aussparung
6: proximales Ende
7: Mantelfläche
8: Hülsenelement
9: Führungskulisse
10: Innendurchmesser
11: Austrittsöffnung
12: distales Ende des Hülsenelements
13: Ausnehmung
14: Ausnahmebereich
15: Kappenelement
16: Berandung
17: Federelement
18: Stechmittel
19: Flügelelement
20: Stechmittelschutzeinrichtung
21: Rippen
22: Ausbuchtungen
23: Ausgangsstellung
24: Endposition

## Patentansprüche

1. Sicherheitsvorrichtung (2) für Spritzen mit einem Spritzenkörper (1) und einem an dem distalen Ende des Spritzenkörpers (1) angeordneten Stechmittel (18), die Sicherheitseinrichtung umfassend ein sich entlang einer axialen Richtung erstreckendes Hülsenelement (8), welches vorgesehen ist, zumindest teilweise das Stechmittel (18) und den Spritzenkörper (1) zu umschliessen, und ein Kragenelement (3), welches an einem distalen Endbereich des Spritzenkörpers (1) anordenbar ist und die Sicherheitsvorrichtung (2) in axialer Richtung arretiert, wobei das Kragenelement (3) zumindest einen Führungsstift (4) aufweist, welcher in zumindest einer Führungskulisse (9) des Hülsenelements (8) eingreift, wobei die Sicherheitsvorrichtung (2) ein Kappenelement (15) aufweist, welches zumindest abschnittsweise über dem Hülsenelement (8) anordenbar ist und durch welches das Hülsenelement (8) bezüglich einer Relativbewegung des Spritzenkörpers (1) zu dem Hülsenelement (8) arretierbar ist, wobei das Kragenelement (3) vorgesehen ist, in einer Umfangsrichtung rotierbar an dem distalen Endbereich des Spritzenkörpers (1) angeordnet zu sein, wobei das Kappenelement (15) eine Stechmittelschutzeinrichtung (20) aufweist, in welcher das Stechmittel (18) anordenbar ist und welche mit dem Kragenelement (3) in Wirkkontakt bringbar ist, wodurch das Kragenelement (3) bezüglich der Rotation arretierbar ist, wobei das Hülsenelement (8) und das Kragenelement (3) jeweils mit einem Verrastmechanismus vorgesehen ist, **dadurch gekennzeichnet, dass** der Verrastmechanismus des Hülsenelementes (8) zumindest eine Rippe (21) oder Welle aufweist, die vorgesehen ist, longitudinal entlang der Länge des Spritzenkörpers (1) angeordnet zu sein.

2. Sicherheitsvorrichtung (2) nach Anspruch 1, **dadurch gekennzeichnet, dass** das Hülsenelement (8) zumindest zwei gegenüberliegende Rippen (21) oder Wellen aufweist.

3. Sicherheitsvorrichtung (2) nach Anspruch 1 oder 2, **dadurch gekennzeichnet, dass** die Rippen (21) oder Wellen Flanken mit unterschiedlichen Flankenwinkeln aufweisen, wobei der eine Flankenwinkel flach und der andere Flankenwinkel steil ausgebildet ist.

4. Sicherheitsvorrichtung (2) nach einem der vorhergehenden Ansprüche, **dadurch gekennzeichnet, dass** sich am distalen Ende (12) eine Austrittsöffnung (11) befindet und das proximale Ende vorgesehen ist, mit dem Spritzenkörper (1) verbunden zu sein.

5. Sicherheitsvorrichtung (2) nach einem der vorhergehenden Ansprüche, **dadurch gekennzeichnet, dass** der Verrastmechanismus des Kragenelements (3) zumindest eine Ausbuchtung (22) aufweist, wobei die Ausbuchtung Flanken mit unterschiedlichen Flankenwinkeln aufweist, und der eine Flankenwinkel flach und der andere Flankenwinkel steil ausgebildet ist.

6. Sicherheitsvorrichtung (2) nach Anspruch 5, **dadurch gekennzeichnet, dass** das Kragenelement (3) zumindest zwei gegenüberliegende Ausbuchtungen aufweist.

7. Sicherheitsvorrichtung (2) nach einem der vorhergehenden Ansprüche, **dadurch gekennzeichnet, dass** der Innendurchmesser des Hülsenelementes (8) und der Außendurchmesser des Kragenelementes (3) sich in den Bereichen der Ausbuchtungen des Kragenelementes (3) und der Rippen (21) des Hülsenelementes (8) überdecken.

8. Sicherheitsvorrichtung (2) nach einem der vorhergehenden Ansprüche, **dadurch gekennzeichnet, dass** vorgesehen ist, dass das Hülsenelement (8) und das Kragenelement (3) an den Rippen (21) und Ausbuchtungen nur in eine Richtung zueinander rotieren.

9. Sicherheitsvorrichtung (2) nach einem der vorhergehenden Ansprüche, **dadurch gekennzeichnet, dass** ein Bereich des Außendurchmessers des Kragenelementes (3) durch den Innendurchmesser des Hülsenelementes (8) nicht überdeckt wird, so dass die Rippen (21) des Hülsenelementes (8) freistehen.

10. Sicherheitsvorrichtung (2) nach einem der vorhergehenden Ansprüche, **dadurch gekennzeichnet, dass** die Sicherheitsvorrichtung (2) mindestens ein Federelement (17) aufweist, das vorgesehen ist, mit dem Spritzenkörper (1) wirkverbunden zu sein und der Relativbewegung des Hülsenelementes (8) zu der Sicherheitsvorrichtung (2) entgegenwirkt, wobei das Kragenelement (3) vorgesehen ist, beim Drücken gegen die Federkraft durch die Sicherheitsvorrichtung (2) aufgrund der vorgegebenen Führungskulisse (9) zu rotieren und über die Rippen (21) des Hülsenelementes (8) zu schnappen.

11. Sicherheitsvorrichtung (2) nach einem der vorhergehenden Ansprüche, **dadurch gekennzeichnet, dass** aufgrund der Flankenwinkel und des Verrastmechanismus des Hülsenelementes (8) und des Kragenelementes (3) eine Rotation des Kragenelementes (3) in dem Bereich der Führungskulisse (9), der in eine Ausgangsstellung führt, ausgeschlossen ist.

12. Sicherheitsvorrichtung (2) nach einem der vorhergehenden Ansprüche, **dadurch gekennzeichnet, dass** sich das Kragenelement (3) in der Ausgangsstellung an einem proximalen Ende des Hülsenelementes (8) befindet und sich die Austrittsöffnung an einem distalen Ende des Hülsenelementes (8) befindet.

13. Sicherheitsvorrichtung (2) nach einem der vorhergehenden Ansprüche, **dadurch gekennzeichnet, dass** das Kragenelement (3) in dem Bereich der Führungskulisse (9) gehalten wird, der in der Arretierung des Kragenelementes (3) zum Hülsenelement (8) ausläuft.

14. Verfahren zur Montage einer Sicherheitsvorrichtung (2) nach einem der Ansprüche 1 bis 13, auf einem Spritzenkörper (1), umfassend die Schritte:
a. Bereitstellen eines Hülsenelements (8), eines Federelements (17) und eines Kragenelements (3),
b. Einbringen des Federelements in das Innere des Hülsenelements entlang einer Montagerichtung,
c. Einbringen des Kragenelementes (3) in das Innere des Hülsenelements entlang der Montagerichtung,
d. Erhalten der Sicherheitseinrichtung,
e. Montieren der Sicherheitseinrichtung auf dem Spritzenkörper (1) durch Verbinden des Kragenelements (3) mit dem Spritzenkörper (1); wobei das Kragenelement (3) zumindest einen Führungsstift (4) aufweist, welcher in zumindest einer Führungskulisse (9) des Hülsenelements (8) eingreift, wobei die Sicherheitsvorrichtung (2) ein Kappenelement (15) aufweist, welches zumindest abschnittsweise über dem Hülsenelement (8) anordenbar ist und durch welches das Hülsenelement (8) bezüglich der Relativbewegung des Spritzenkörpers (1) zu dem Hülsenelement (8) arretierbar ist, wobei das Kragenelement (3) in einer Umfangsrichtung rotierbar an dem distalen Endbereich des Spritzenkörpers (1) angeordnet ist, wobei das Kappenelement (15) eine Stechmittelschutzeinrichtung (20) aufweist, in welcher das Stechmittel (18) anordenbar ist und welche mit dem Kragenelement (3) in Wirkkontakt bringbar ist, wodurch das Kragenelement (3) bezüglich der Rotation arretierbar ist, wobei das Hülsenelement (8) und das Kragenelement (3) jeweils mit einem Verrastmechanismus vorgesehen ist, **dadurch gekennzeichnet, dass** der Verrastmechanismus des Hülsenelementes (8) zumindest eine Rippe (21) oder Welle aufweist, die longitudinal entlang der Länge des Spritzenkörpers (1) angeordnet ist.

15. Spritzensicherungssystem aufweisend eine Sicherungsvorrichtung nach einem der Ansprüche 1 bis 13.

16. Spritze ausgestattet mit einer Sicherungsvorrichtung nach einem der Ansprüche 1 bis 13.

## Claims

1. Safety apparatus (2) for syringes that have a syringe body (1) and have a piercing means (18) arranged at the distal end of the syringe body (1), the safety device comprising a sleeve element (8), which extends along an axial direction and is intended to at least partially enclose the piercing means (18) and the syringe body (1), and a collar element (3), which is arrangeable at a distal end region of the syringe body (1) and arrests the safety apparatus (2) in the axial direction, wherein the collar element (3) has at least one guide pin (4) which engages in at least one guide slot (9) of the sleeve element (8), wherein the safety apparatus (2) has a cap element (15) which is arrangeable over the sleeve element (8) at least sectionally and by way of which the sleeve element (8) is arrestable with regard to a relative movement of the syringe body (1) with respect to the sleeve element (8), wherein the collar element (3) is intended to be arranged at the distal end region of the syringe body (1) so as to be rotatable in a circumferential direction, wherein the cap element (15) has a piercing-means-protection device (20) in which the piercing means (18) is arrangeable and which is able to be brought into operative contact with the collar element (3), whereby the collar element (3) is arrestable with regard to the rotation, wherein the sleeve element (8) and the collar element (3) are each provided with a latching mechanism, **characterized in that** the latching mechanism of the sleeve element (8) has at least one rib (21) or corrugation that is intended to be arranged longitudinally along the length of the syringe body (1).

2. Safety apparatus (2) according to Claim 1, **characterized in that** the sleeve element (8) has at least two oppositely situated ribs (21) or corrugations.

3. Safety apparatus (2) according to Claim 1 or 2, **characterized in that** the ribs (21) or corrugations have flanks with different flank angles, wherein one flank angle is shallow and the other flank angle is steep.

4. Safety apparatus (2) according to one of the preceding claims, **characterized in that** an exit opening (11) is situated at the distal end (12) and the proximal end is intended to be connected to the syringe body (1).

5. Safety apparatus (2) according to one of the preceding claims, **characterized in that** the latching mechanism of the collar element (3) has at least one protrusion (22), wherein the protrusion has flanks with different flank angles, and one flank angle is shallow and the other flank angle is steep.

6. Safety apparatus (2) according to Claim 5, **characterized in that** the collar element (3) has at least two oppositely situated protrusions.

7. Safety apparatus (2) according to one of the preceding claims, **characterized in that** the inner diameter of the sleeve element (8) and the outer diameter of the collar element (3) overlap in the regions of the protrusions of the collar element (3) and of the ribs (21) of the sleeve element (8).

8. Safety apparatus (2) according to one of the preceding claims, **characterized in that** provision is made for the sleeve element (8) and the collar element (3) to rotate with respect to one another only in one direction at the ribs (21) and protrusions.

9. Safety apparatus (2) according to one of the preceding claims, **characterized in that** a region of the outer diameter of the collar element (3) is not overlapped by the inner diameter of the sleeve element (8), such that the ribs (21) of the sleeve element (8) are free.

10. Safety apparatus (2) according to one of the preceding claims, **characterized in that** the safety apparatus (2) has at least one spring element (17) which is intended to be connected operatively to the syringe body (1) and which counteracts the relative movement of the sleeve element (8) with respect to the safety apparatus (2), wherein the collar element (3) is intended to rotate, and to snap over the ribs (21) of the sleeve element (8), by way of the safety apparatus (2) on account of the predefined guide slot (9) when pressed counter to the spring force.

11. Safety apparatus (2) according to one of the preceding claims, **characterized in that**, owing to the flank angles and to the latching mechanism of the sleeve element (8) and of the collar element (3), a rotation of the collar element (3) is ruled out in the region of the guide slot (9) that leads into a starting position.

12. Safety apparatus (2) according to one of the preceding claims, **characterized in that** the collar element (3) is situated in the starting position at a proximal end of the sleeve element (8) and the exit opening is situated at a distal end of the sleeve element (8).

13. Safety apparatus (2) according to one of the preceding claims, **characterized in that** the collar element (3) is held in the region of the guide slot (9) that ends in the arresting of the collar element (3) with respect to the sleeve element (8).

14. Method for mounting a safety apparatus (2) according to one of Claims 1 to 13 on a syringe body (1), comprising the steps of:
a. providing a sleeve element (8), a spring element (17) and a collar element (3),
b. introducing the spring element into the interior of the sleeve element along a mounting direction,
c. introducing the collar element (3) into the interior of the sleeve element along the mounting direction,
d. obtaining the safety device,
e. mounting the safety device on the syringe body (1) by connecting the collar element (3) to the syringe body (1); wherein the collar element (3) has at least one guide pin (4) which engages in at least one guide slot (9) of the sleeve element (8), wherein the safety apparatus (2) has a cap element (15) which is arrangeable over the sleeve element (8) at least sectionally and by way of which the sleeve element (8) is arrestable with regard to the relative movement of the syringe body (1) with respect to the sleeve element (8), wherein the collar element (3) is arranged at the distal end region of the syringe body (1) so as to be rotatable in a circumferential direction, wherein the cap element (15) has a piercing-means-protection device (20) in which the piercing means (18) is arrangeable and which is able to be brought into operative contact with the collar element (3), whereby the collar element (3) is arrestable with regard to the rotation, wherein the sleeve element (8) and the collar element (3) are each provided with a latching mechanism, **characterized in that** the latching mechanism of the sleeve element (8) has at least one rib (21) or corrugation that is arranged longitudinally along the length of the syringe body (1).

15. Syringe safety system having a safety apparatus according to one of Claims 1 to 13.

16. Syringe equipped with a safety apparatus according to one of Claims 1 to 13.

## Revendications

1. Dispositif de sécurité (2) pour seringues, comprenant un corps de seringue (1) et un moyen de perçage (18) disposé à l'extrémité distale du corps de seringue (1), le dispositif de sécurité comprenant un élément de douille (8) s'étendant le long d'une direction axiale, qui est prévu pour entourer au moins en partie le moyen de perçage (18) et le corps de seringue (1), et un élément de collerette (3) qui peut être disposé sur une zone d'extrémité distale du corps de seringue (1) et bloque le dispositif de sécurité (2) dans la direction axiale, dans lequel l'élément de collerette (3) présente au moins une goupille de guidage (4) qui vient en prise dans au moins une coulisse de guidage (9) de l'élément de douille (8), dans lequel le dispositif de sécurité (2) présente un élément de capuchon (15) qui peut être disposé au moins par endroits sur l'élément de douille (8) et qui permet de bloquer l'élément de douille (8) concernant un mouvement relatif du corps de seringue (1) par rapport à l'élément de douille (8), dans lequel l'élément de collerette (3) est prévu pour être disposé en rotation dans une direction circonférentielle au niveau de la zone d'extrémité distale du corps de seringue (1), dans lequel l'élément de capuchon (15) présente un dispositif de protection de moyen de perçage (20) dans lequel le moyen de perçage (18) peut être disposé et qui peut être mis en contact actif avec l'élément de collerette (3), de sorte que l'élément de collerette (3) peut être bloqué au niveau de la rotation, l'élément de douille (8) et l'élément de collerette (3) étant respectivement munis d'un mécanisme d'enclenchement, **caractérisé en ce que** le mécanisme d'enclenchement de l'élément de douille (8) présente au moins une nervure (21) ou un arbre qui est prévu(e) pour être disposé(e) de manière longitudinale le long de la longueur du corps de seringue (1).

2. Dispositif de sécurité (2) selon la revendication 1, **caractérisé en ce que** l'élément de douille (8) présente au moins deux nervures (21) ou arbres opposés.

3. Dispositif de sécurité (2) selon la revendication 1 ou 2, **caractérisé en ce que** les nervures (21) ou arbres présentent des flancs ayant différents angles de flanc et un angle de flanc est plat et l'autre angle de flanc est raide.

4. Dispositif de sécurité (2) selon l'une quelconque des revendications précédentes, **caractérisé en ce qu'**une ouverture de sortie (11) se trouve à l'extrémité distale (12) et l'extrémité proximale est prévue pour être reliée au corps de seringue (1).

5. Dispositif de sécurité (2) selon l'une quelconque des revendications précédentes, **caractérisé en ce que** le mécanisme d'enclenchement de l'élément de collerette (3) présente au moins une échancrure (22), l'échancrure présentant des flancs ayant différents angles de flanc, et un angle de flanc est plat et l'autre angle de flanc est raide.

6. Dispositif de sécurité (2) selon la revendication 5, **caractérisé en ce que** l'élément de collerette (3) présente au moins deux échancrures opposées.

7. Dispositif de sécurité (2) selon l'une quelconque des revendications précédentes, **caractérisé en ce que** le diamètre intérieur de l'élément de douille (8) et le diamètre extérieur de l'élément de collerette (3) se chevauchent dans les zones des échancrures de l'élément de collerette (3) et des nervures (21) de l'élément de douille (8).

8. Dispositif de sécurité (2) selon l'une quelconque des revendications précédentes, **caractérisé en ce qu'**il est prévu que l'élément de douille (8) et l'élément de collerette (3) ne tournent que dans un seul sens l'un par rapport à l'autre au niveau des nervures (21) et des échancrures.

9. Dispositif de sécurité (2) selon l'une quelconque des revendications précédentes, **caractérisé en ce qu'**une zone du diamètre extérieur de l'élément de collerette (3) n'est pas recouverte par le diamètre intérieur de l'élément de douille (8) de sorte que les nervures (21) de l'élément de douille (8) sont exposées.

10. Dispositif de sécurité (2) selon l'une quelconque des revendications précédentes, **caractérisé en ce que** le dispositif de sécurité (2) présente au moins un élément faisant ressort (17) qui est prévu pour être en relation active avec le corps de seringue (1) et qui contre le mouvement relatif de l'élément de douille (8) par rapport au dispositif de sécurité (2), dans lequel l'élément de collerette (3) est prévu, lorsque le dispositif de sécurité (2) pousse contre la force ressort, pour tourner en raison de la coulisse de guidage (9) spécifiée et s'enclencher au-delà des nervures (21) de l'élément de douille (8).

11. Dispositif de sécurité (2) selon l'une quelconque des revendications précédentes, **caractérisé en ce qu'**en raison des angles de flanc et du mécanisme d'enclenchement de l'élément de douille (8) et de l'élément de collerette (3), une rotation de l'élément de collerette (3) dans la zone de la coulisse de guidage (9) menant à une position initiale est exclue.

12. Dispositif de sécurité (2) selon l'une quelconque des revendications précédentes, **caractérisé en ce que** l'élément de collerette (3) se trouve dans la position initiale à une extrémité proximale de l'élément de douille (8) et l'ouverture de sortie se trouve à une extrémité distale de l'élément de douille (8).

13. Dispositif de sécurité (2) selon l'une quelconque des revendications précédentes, **caractérisé en ce que** l'élément de collerette (3) est maintenu dans la zone de la coulisse de guidage (9) qui se termine au niveau du blocage de l'élément de collerette (3) vers l'élément de douille (8).

14. Procédé de montage d'un dispositif de sécurité (2) selon l'une quelconque des revendications 1 à 13, sur un corps de seringue (1), comprenant les étapes consistant à :
a) fournir un élément de douille (8), un élément formant ressort (17) et un élément de collerette (3),
b) introduire l'élément faisant ressort à l'intérieur de l'élément de douille le long d'un sens de montage,
c) introduire l'élément de collerette (3) à l'intérieur de l'élément de douille le long du sens de montage,
d) obtenir le dispositif de sécurité,
e) monter le dispositif de sécurité sur le corps de seringue (1) en reliant l'élément de collerette (3) au corps de seringue (1) ; dans lequel l'élément de collerette (3) présente au moins une goupille de guidage (4) qui vient en prise dans au moins une coulisse de guidage (9) de l'élément de douille (8), dans lequel le dispositif de sécurité (2) présente un élément de capuchon (15) qui peut être disposé au moins par endroits sur l'élément de douille (8) et qui permet de bloquer l'élément de douille (8) concernant le mouvement relatif du corps de seringue (1) par rapport à l'élément de douille (8), dans lequel l'élément de collerette (3) est disposé en rotation dans une direction circonférentielle au niveau de la zone d'extrémité distale du corps de seringue (1), dans lequel l'élément de capuchon (15) présente un dispositif de protection de moyen de perçage (20) dans lequel le moyen de perçage (18) peut être disposé et qui peut être mis en contact actif avec l'élément de collerette (3), de sorte que l'élément de collerette (3) peut être bloqué au niveau de la rotation, dans lequel l'élément de douille (8) et l'élément de collerette (3) sont respectivement munis d'un mécanisme d'enclenchement, **caractérisé en ce que** le mécanisme d'enclenchement de l'élément de douille (8) présente au moins une nervure (21) ou un arbre qui est disposé(e) de manière longitudinale le long de la longueur du corps de seringue (1).

15. Système de sécurité de seringue présentant un dispositif de sécurité selon l'une quelconque des revendications 1 à 13.

16. Seringue, équipée d'un dispositif de sécurité selon l'une quelconque des revendications 1 à 13.
